# EUROPEAN PATENT SPECIFICATION

(11) **EP 1 492 580 B1**
(45) Date of publication and mention of the grant of the patent: **13.06.2012**
(21) Application number: 03711039.2
(22) Date of filing: 13.02.2003
(51) Int. Cl.: A61L 31/10, A61L 31/12, A61L 31/14

(54) **BIODEGRADABLE STENTS WITH CONTROLLED CHANGE OF PHYSICAL PROPERTIES LEADING TO BIOMECHANICAL COMPATIBILITY**
BIOLOGISCH ABBAUBARER STENT MIT VERÄNDERTEN PHYSIKALISCHEN EIGENSCHAFTEN ZUR ERHÖHUNG DER BIOKOMPATIBILITÄT
STENTS BIODEGRADABLES DONT LA MODIFICATION COMMANDEE DES PROPRIETES PHYSIQUES DEBOUCHE SUR UNE COMPATIBILITE BIOMECANIQUE

(30) Priority: 14.02.2002 US 75970
(43) Date of publication of application: 05.01.2005
(73) Proprietor: Boston Scientific Limited, St. Michael (BB)
(72) Inventor: HELMUS, Michael, Worcester, MA 01609 (US)
(74) Representative: Hauck Patent- und Rechtsanwälte
(86) International application number: PCT/US2003/004443
(87) International publication number: WO 2003/068288

(56) References cited:
- EP-A- 1 110 561
- EP-A- 1 112 724
- WO-A-00/40278
- US-A- 6 139 510

## Description

### FIELD OF THE INVENTION

The present invention relates to implantable or insertable medical devices and, in particular, to implantable or insertable medical devices such as intraluminal stents, at least a portion of which biodegrades or bioresorbs *in vivo.*

### BACKGROUND OF THE INVENTION

Biodegradable implantable or insertable medical devices such as intraluminal stents including, e.g., coronary vascular stents, urethral, ureteral, biliary, tracheal, gastrointestinal and esophageal stents are generally known. Such biodegradable stents are typically manufactured from polymeric materials including, e.g., polylactic acid (PLA), polyglycolic acid (PGA), polycaprolactone (PCA) as well as copolymers and composites thereof.

Biodegradable medical devices can provide distinct advantages relative to non-biodegradable medical devices. While some medical devices are intended to remain permanently implanted, this is often not necessary or desirable. Permanently implanted medical devices must be constructed of non-biodegradable materials and such materials may not be as biocompatible with the surrounding and contacting tissue as are biodegradable materials. The lack of biocompatibility and the extended presence of such a device in the body may result in both localized damage to the surrounding tissue and systemic complications. Damage can also result if a permanently implantable medical device is not biomechanically compatible with the contacting tissue, i.e., the device must possess mechanical properties such as strength and flexibility that are similar to those of the contacting tissue.

For example, in the case of a coronary stent inserted after percutaneous transluminal coronary angioplasty ("PCTA"), the extended presence of a non-biodegradable stent can exacerbate neointimal hyperplasia which is believed to be a significant causative factor in the restenosis of the vessel. A completely biodegradable coronary stent would to some extent, therefore, minimize the complications associated with the presence of a permanent device and would obviate the need to remove the stent after it has served its intended function.

Notwithstanding the foregoing advantages generally associated with the use of biodegradable medical devices, there exist distinct disadvantages with such devices that are designed to biodegrade in vivo. Among such disadvantages include the premature loss of mechanical strength of the device. For example, in the case of an intravascular stent such as a coronary stent commonly used to prevent acute collapse of a coronary vessel after PCTA and to decrease restenosis of the vessel, the loss of mechanical strength can result in the failure of the device to maintain the patency of the coronary vessel during the remodeling and healing period.

The loss of mechanical strength associated with biodegradable implantable or insertable medical devices is, in many cases, associated with the relatively uncontrolled rate and/or extent of biodegradation of the material (s) used in the construction of such devices. In many commonly disclosed biodegradable materials such as those mentioned above, biodegradation proceeds by hydrolytic chain cleavage of the polymer (s) upon contact thereof with bodily fluids. Such hydrolysis results in the production of lower molecular weight species that are thus separated from the remaining bulk of the material and which can be further metabolized or resorbed by, or excreted from the body. In the case of a completely biodegradable device, the device is eventually completely biodegraded or resorbed by the body:

As the device biodegrades in vivo, the device loses mass and can prematurely lose mechanical strength. Significant premature loss in mechanical strength of the device can, as discussed above, render the device incapable of performing its intended function, e. g., to maintain the patency of a body lumen such as a coronary vessel for a period of time. This period of time is generally the period during which the lumen or vessel undergoes healing and remodeling or otherwise does not possess the necessary mechanical properties to remain patent without the support of the medical device. In addition, the loss of mechanical strength of the device can cause fragmentation of the device into pieces that can occlude the body lumen or damage the surrounding tissue.
EP 1 112 724 A2 discloses a removable stent for body lumens made from a soft, flexible fiber having an outer surface. An outer bioabsorbable/degradable coating is applied to the outer surface of the filament causing it to become rigid. The coating softens in vivo through absorption and/or degradation such that the stent is readily passed or removed from the lumen as a softened filament after a predetermined period of time through normal flow of body fluids passing through the lumen or by manual removal.
EP 1 110 561 A2 discloses a biodegradable stent having an inner core and an outer layer. The inner core has a first degradation rate, and the outer layer has a second degradation rate which is slower than the degradation rate of the inner core.
US 6,139,510 discloses an super elastic alloy guidewire made of a high elasticity metal alloy, preferably a Ni-Ti alloy. The guidewire is coated with lubricious polymer to enhance its suitability for use within a catheter and with the interior of vascular lumen.

The problem of the invention is to provide an implantable or insertable medical device, at least a portion of which is biodegradable, wherein after insertion or implantation into a patient, the medical device becomes decreasingly rigid and increasingly biomechanically compatible with body tissue in contact with the device over time. Such a device would ideally, therefore, substantially maintain its mechanical properties for a time period upon being inserted or implanted and thereafter, in a controlled and predictable manner, become increasingly biomechanically compatible with the contacting luminal walls.
The problem is solved by a medical device according to claims 1.

### SUMMARY OF THE INVENTION

In a first embodiment, the present invention provides an implantable or insertable medical device comprising a inner, or core, material and a biodegradable coating or covering material at least partially covering the inner, or core, material; wherein after insertion or implantation into a patient, the medical device becomes decreasingly rigid and increasingly biomechanically compatible with body tissue in contact with the device over time. The core is a multifilament core comprising a composite of non-biodegradable wires or filaments and biodegrabable wires or filaments.

In the present invention in which the core material become more flexible, the coating material preferably substantially controls the rate at which the core material becomes flexible upon contact with body fluids. In such embodiments, it is particularly preferred that the coating material is a hydrophobic surface erodable polymer.

It is generally preferred that at least one of the core material and coating material and, more preferably, both the core material and coating material, be polymers. Biodegradable polymers include shape memory biodegradable polymers. Additional coating layers may also be provided on the medical devices of the present invention.

In specific embodiments of the present invention either or both of the core material or coating material and/or any additional coating layer may contain therein or thereon at least one therapeutic. The therapeutic agent is provided to be released from the medical device at the location where the device is implanted and may provide both local and systemic therapeutic benefit.

Preferred medical devices of the present invention include intraluminal stents, such as stents selected from the group consisting of coronary, biliary, urethral, ureteral, tracheal, gastrointestinal and esophageal stents. Particularly preferred medical devices of the present invention are coronary stents, which may be self-expandable or balloon- expandable.

### DETAILED DESCRIPTION OF THE INVENTION

The medical devices of the present invention, for example, intraluminal stents, when initially implanted at a desired location in the body, have mechanical properties such as strength and flexibility that, for example, are suitable for scaffolding and maintaining the patency of the lumen. A coronary intravascular stent, for instance, is commonly used to scaffold a coronary artery that has undergone percutaneous transluminal coronary angioplasty (PCTA).

PCTA, which is beneficially used to dilate an obstructed blood vessel by compressing arterial plaque against the walls of the vessel, also traumatizes the vessel.
The traumatized vessel is, consequently, susceptible to abrupt reclosure which is believed to arise in part from elastic recoil of the traumatized vessel. Abrupt reclosure occurs generally within hours to a few days following the angioplasty procedure and can lead to myocardial infarction and even death.

There also exists a substantial risk that a blood vessel that has undergone angioplasty will renarrow, a phenomenon known as "restenosis." Restenosis is believed to be caused by the proliferation of smooth muscle cells, i.e., "neointimal hyperplasia," associated with the "remodeling" of the vessel after angioplasty. Intravascular coronary stents provided with a therapeutic agent for localized release at the site of vessel trauma, such as an anti-proliferative or antithrombotic agent, can be particularly advantageous in preventing restenosis of an occluded vessel and clot formation. The occurrence of restenosis is particularly prevalent during the first three to six months following angioplasty.

The scaffolding function of intraluminal stents is also of importance, particularly in the case of an intravascular coronary stent, during the initial three to six months following implantation. It is during this period that the lumen is most likely to lack the inherent mechanical strength to remain patent without the support provided by the stent. Subsequent to this initial period of healing and remodeling, the lumen is much more likely to remain patent with either no support provided by a stent or other medical device, or with less support than is initially required upon implantation of the device.

Consequently, it is desirable that the device, when initially implanted, be able to provide a greater level of support for the lumen walls than is required after substantial healing has occurred. This greater level of support necessitates providing, at least initially, a relatively more rigid device that is consequently less compliant or biomechanically compatible with the lumen wall tissue.

More particularly, although the increased rigidity of the device is beneficial during the primary healing stage, it can become problematic after the lumen wall tissue has healed and remodeled sufficiently to possess the inherent mechanical strength to remain patent either with no scaffolding or with decreased support provided by an intraluminal stent.

To the extent that the stent remains relatively rigid after substantial healing and remodeling of the lumen wall, the stent will often be undesirably less compliant than the lumen wall, i.e., less biomechanically compatible with lumen wall. This lack of biomechanical compatibility of the device with the lumen wall can lead to irritation of the vessel wall and, consequently, to restenosis of the same.

The implantable or insertable medical device of the present invention is adapted to provide a controlled change in its biomechanical compatibility with the luminal walls. To achieve this objective, the medical device is constructed in a manner such that it substantially maintains, for a time period, its mechanical properties, particularly its rigidity relative to the luminal walls upon being initially inserted or implanted. Thereafter, the medical becomes increasingly biomechanically compatible with the contacting tissue surfaces such as the luminal walls.

This controlled change in mechanical properties exhibited by the medical device after being implanted or inserted advantageously allows the medical device to provide different levels of scaffolding, depending upon the time-dependent needs of the contacting luminal walls. Thus, the medical device is adapted to provide an initial level of scaffolding that may be required for a first time period during which the luminal walls undergo remodeling and healing. During this period, it may be desirable for the device to remain relatively rigid to support the luminal walls that otherwise do not possess the mechanical properties to remain patent without such support. In the case of an intravascular coronary stent, the relatively rigid mechanical properties possessed by the stent for the first time period can help to prevent abrupt reclosure and restenosis of the vessel. During this first time period, at least a portion of the device is adapted to biodegrade or bioresorb while still providing the necessary mechanical properties to support the luminal walls.

After this first time period during which remodeling and healing occurs, the medical device becomes increasingly biomechanically compatible with the surrounding and contacting tissue. Thus, in the case of an intraluminal stent, the stent will then possess mechanical properties that are consistent with the healed and remodeled luminal walls.

While the medical device of the present of the present invention remains relatively rigid for an initial period of time, the present invention is not to be construed as limited to medical devices which necessarily exhibit a sharp, abrupt, or discontinuous change in flexibility. Medical devices that continuously become increasingly flexible, i.e., biomechanically compatible, but still maintain, for a period of time, sufficient rigidity to support the luminal walls that otherwise do not possess the mechanical properties to remain patent without such support are included within the scope of the present invention.

The thus compliant medical device can then remain indefinitely implanted, i.e., undergo no further biodegradation, or the device can continue to undergo biodegradation while nonetheless remaining compliant, until the device is eventually completely biodegraded by the body.

Thus, the device exists in compliant form, whether it eventually completely biodegrades or remains permanently implanted, after the first time period following implantation during which it must often be relatively more rigid. As such, the device is considerably less likely to irritate or damage the luminal walls and/or macroscopically fragment into pieces that can occlude the lumen or damage the surrounding tissue.

The medical device in accordance with the present invention, which is adapted to provide a controlled change in its mechanical properties upon being inserted or implanted, comprises an inner or core material and a biodegradable coating or covering material at least partially covering the core material. The core material may either be biodegradable or non-biodegradable.

Where the core material and the coating material are both biodegradable, the medical device will, eventually, be completely biodegraded. However, prior to complete biodegradation, the device will undergo a controlled change in its mechanical properties as herein described, rendering it increasingly biomechanically compatible, i.e., "compliant," with the surrounding and contacting tissue. Where only the coating material is biodegradable and the core material is substantially non-biodegradable, the medical device of the present invention will only be partially biodegraded. In this embodiment, the device will again undergo a controlled change in its mechanical properties as herein described, rendering the remaining structure increasingly more biomechanically compatible, i.e., more compliant with the surrounding and contacting tissue. In this embodiment, the remaining structure will remain implanted for an indefinite period of time, after which it may be surgically removed if desired.

Any portion of a medical device of the present invention that is described herein as "biodegradable," "bioresorbable," or "bioerodable" will, over time, lose bulk mass by being degraded, resorbed or eroded by normal biological processes in the body. As used herein, the term "biodegradable" is intended to encompass the terms "bioresorbable" and "bioerodable." Typically, the material is metabolized or broken down by normal biological processes into metabolites or break-down products that are substantially non-toxic to the body and are capable of being resorbed and/or eliminated through normal excretory and metabolic processes of the body. Such biological processes include those that are primarily mediated by metabolic routes such as enzymatic action or by simple hydrolytic action under normal physiological pH conditions.

In addition, the description herein will, at times, refer to "surface erodable" and "bulk erodable" biodegradable materials. Surface erodable materials are materials in which bulk mass is lost primarily at the surface of the material that is in direct contact with the physiologic environment, such as body fluids. Bulk erodable materials are materials in which bulk mass is lost throughout the mass of the material, i.e., loss of bulk mass is not limited to mass loss that occurs primarily at the surface of the material in direct contact with the physiological environment.

In accordance with some embodiments of the invention, the core is either initially flexible (e.g., with a rigid biodegradable coating or covering material at least partially covering the core) or becomes more flexible over time. For example, the core can become more flexible upon contact with body fluids, which can control metabolic, hydrolytic or physical processes by which the core material is rendered more flexible. Thus, a core material that is initially less flexible than the coating material may be rendered more flexible by undergoing limited biodegradation or, more preferably, simply by absorbing body fluids.

In accordance with this embodiment of the present invention, in which the core portion is more flexible than the coating material or becomes more flexible upon contact with body fluids, the device can be adapted to provide mechanical properties that exhibit a controlled change from, for example, an initially more rigid device, to one that is increasingly more biomechanically compatible with the surrounding and contacting tissue. Such a device can thus initially provide a greater level of scaffolding to, for example, a coronary artery that has undergone PCTA for an initial time period, preferably three to six months, more preferably from about two to about four months, wherein complications such as abrupt reclosure or restenosis are particularly prevalent. Thereafter, the device, having adopted or assumed mechanical properties more biomechanically compatible or compliant with the substantially remodeled or healed tissue, can remain in place as a permanent implantation or biodegrade. In either case, the medical device of the present invention provides a decreased risk of tissue damage caused by lack of biomechanical compatibility and/or the possibility of fragmentation. In the case of a coronary vascular stent in accordance with the present invention, because restenosis is believed to be associated with the lack of biomechanical compatibility between the stent and the surrounding and contacting tissue, such a stent provides a decreased risk of restenosis.

Where the core material is initially relatively inflexible, it desirable that the coating material be selected such that it substantially controls the rate at which the core material becomes more flexible. Coating materials that control the penetration of body fluids to the core are particularly preferable to achieve this objective. Among such coating materials are those that provide a diffusion barrier that substantially prevents body fluids from contacting the core material until the coating material has substantially biodegraded.

Such preferred coating materials are those that are surface erodable materials, including surface erodable polymeric materials. Hydrophobic surface erodable polymers are particularly preferred in this embodiment of the present invention. Surface erodable polymeric materials are those, as described above, in which bulk mass loss occurs primarily at the surface of the material rather than throughout the bulk of the coating material. By confining bulk mass loss to primarily the surface, the coating material acts a barrier to penetration of body fluids that can further penetrate into the core material and cause it to prematurely become more flexible. Thus, the medical device of the present invention in which the coating material acts as a diffusion barrier preventing the core from become prematurely flexible, allows the medical device to maintain its mechanical properties for a time period after which, penetration of body fluids into the core renders it more flexible and biomechanically compatible.

Among preferred surface erodable polymers useful as coating materials in the present invention include, but are not limited to, polyamides, polyorthoesters and polyanhydrides (PAN), polycaprolactone (PCL), maleic anhydride copolymers, and polyhydroxybutyrate copolymers, as well as mixtures and blends thereof. Polyanhydrides include, e.g., poly 1,3-(bis(p-carbophenoxy)propane anhydride (p-CPP) (an aromatic polyanhydride); the polymer formed from the copolymerization of p-CPP with sebacic acid (a copolymer of an aromatic diacid and an aliphatic diacid); and polyterephthalic acid (i.e., polyterephthalic anhydride, an aromatic anhydride). Surface erodable polymers can be modified to control the rate of surface erosion by, for example, adding basic or acidic additives, depending, for example, on whether degradation primarily proceeds by acid catalyzed or base-catalyzed hydrolysis. Polyorthoesters, for example, degrade primarily by acid catalyzed hydrolysis. Thus, addition of an acidic substance to a polyorthoester can enhance the rate of surface bioerosion. In addition, the thickness of the surface erodable polymer on the core material can be selected to extend or shorten the time period during which erosion occurs and thus extend or shorten the time period prior to substantial penetration of body fluids into the core.

Other biodegradable polymers that are useful in forming the coating material on the core include, but are not limited to poly (L-lactide) (PLLA), poly (D, L-lactide) (PLA), polyglycolide (PGA), poly (L-lactide-co-D, L-lactide) (PLLA/PLA), poly (L-lactide-co-glycolide) (PLLA/PGA), poly (D, L-lactide-co-glycolide) (PLA/PGA), poly (glycolide-co-trimethylene carbonate) (PGA/PTMC), polyethylene oxide (PEO), polydioxanone (PDS), polypropylene fumarate, poly (ethyl glutamate-co-glutamic acid), poly (tert-butyloxy-carbonylmethyl glutamate), polycaprolactone (PCL), polycaprolactone co-butylacrylate, polyhydroxybutyrate (PHUT) and copolymers of polyhydroxybutyrate, poly (phosphazene), poly (D, L-lactide-co-caprolactone) (PLA/PCL), poly (glycolide-co- caprolactone) (PGA/PCL), poly (phosphate ester), poly (amino acid) and poly (hydroxy butyrate), polydepsipeptides, maleic anhydride copolymers, polyphosphazenes, polyiminocarbonates, poly [ (97. 5% dimethyl-trimethylene carbonate)-co- (2. 5% trimethylene carbonate) ], cyanoacrylate, polyethylene oxide, hydroxypropylmethylcellulose, polysaccharides such as hyaluronic acid, chitosan and regenerate cellulose, and proteins such as gelatin and collagen, among others.

In the example, where the core is substantially non-biodegradable, any biocompatible material can be used provided that it is flexible, i.e., biomechanically compatible, or can become flexible, to a level of biomechanical compatibility that renders it compliant with the surrounding and contacting tissue. Thus, the core may become flexible, for example, upon biodegradation of a diffusion barrier coating such as a surface erodable polymer as described above. Any materials conventionally used for implantable or insertable medical devices such as intraluminal stents may thus be utilized for the core provided, as discussed above, that such materials are flexible or can become flexible.

Among such materials that are flexible or can become flexible are included, but are not limited to, biostable macromolecules, e.g., crosslinked collagen; soft polymers, e.g., polyurethane and its copolymers, silicone and its copolymers, polyolefin rubbers, and hydrogels, e.g., polyhydroxyethyl methacrylate, polyvinylpyrrolidone and polyacrylamide. Other suitable polymeric materials include, without limitation, ethylene vinyl-acetate, thermoplastic elastomers, polyvinyl chloride, cellulosics, polyamides, polyesters, polysulfones, polytetrafluoroethylenes, polycarbonates, acrylonitrile butadiene styrene copolymers and acrylic polymers.

The core material may also be comprised of any biocompatible metal or ceramic material generally useful to construct implantable or insertable medical devices including intraluminal stents. Biocompatible ceramic materials useful for forming the core include, but are not limited to, ceramics of alumina and glass-ceramics such as Macor®. Bioactive ceramics and glasses such as Bioglass®, which binds to soft connective tissues and bones, and hydroxyapatites are among other useful biocompatible ceramic materials.

Among useful metallic materials include, but are not limited to, shape memory alloys such as Nitinol®, and other metallic materials including, but not limited to, stainless steel, tantalum, nickel-chrome, or cobalt-chromium, i.e., Elgiloy®. A particularly preferred core material is the superelastic shape memory alloy Nitinol®. Metallic materials that exhibit shape memory characteristics such as Nitinol® are beneficial, *inter alia,* because they allow the intraluminal stent to be configured in a first condition, i.e., an expanded condition, and then shaped at a different temperature to a second condition, i.e., a smaller condition for loading onto a catheter. The intraluminal stent then regains the memorized enlarged shape when warmed to a selected temperature, such as by exposure to human body temperature or by application of an external heat trigger. Shape memory alloys are particularly suitable for use with self-expandable stents, described more fully below. Stents in accordance with the present invention may also be balloon-expandable.

Where metallic materials and other nonbiodegradable materials are used for the core, they are preferably used as thinner gauge materials than when used in conventional intraluminal stents that do not include an outer biodegradable covering material that provides added overall mechanical strength and rigidity to the device.
Thinner gauge core materials provide enhanced flexibility and biomechanical compatibility of the core after biodegradation of the covering material.

Where the medical device is intended to remain indefinitely implanted upon degradation of the covering material, the core can be fabricated from the foregoing metallic materials (or other non-biodegradable materials such as non-biodegradable ceramics or polymers), which may be conventionally shaped as a woven or braided network of multifilaments or wires or as an interconnected network of articulable segments, or, e.g., as a laser-cut or micromachined tube or a coiled structure.
In such multi-filament embodiments, the metallic wires are typically thinner gauge, as discussed above, and may also be braided together with fibers of any of the aforementioned biodegradable materials, including fibers made of surface erodable materials discussed above. In these embodiments, the braided or woven fibers together form the inner or core material over which at least a portion of is provided a biodegradable covering or coating material. According to the invention the core comprises a composite of non-biodegradable wires or filaments and biodegradable wires or filaments, the core can be designed to provide the overall medical device with the requisite mechanical strength and relative rigidity initially necessary to support, e.g., the luminal walls. Upon biodegradation of the non-metallic fibers, the remaining core of flexible metallic wires will be biocompatible and compliant with the surrounding and contacting tissue, leaving a minimum of permanently remaining foreign body.

In the example, in which the entire medical device is biodegradable, i.e., where the device comprises both a biodegradable core material and a biodegradable coating or covering material at least partially covering the core, the device eventually completely biodegrades. In this embodiment, the core may be relatively more flexible than the coating material, or may become more flexible upon, for example, contact with body fluids. In this manner, the device can again be adapted to provide mechanical properties that exhibit a controlled change from, for example, an initially more rigid device, to one that is increasingly biomechanically compatible with the surrounding and contacting tissue. Such a device can then provide a greater level of scaffolding to, for example, a coronary artery that has undergone PCTA for an initial time period, preferably three to six months, more preferably from about two to four months, wherein complications such as abrupt reclosure or restenosis are particularly prevalent.
Thereafter, the device, having adopted or assumed mechanical properties more biomechanically compatible or compliant with the substantially remodeled or healed tissue, can completely biodegrade or bioresorb within a period of preferably from about six months to one year.

The biodegradable core in this example can again soften or become more flexible upon contact with body fluids or it can be constructed of a biodegradable material that is initially more flexible than the relatively more rigid coating or covering layer. In any event, it is preferable that where both the core material and the coating material are biodegradable, the materials be different materials to provide different rates of biodegradation and different mechanical properties. Suitable biodegradable core materials can be any biodegradable polymer, as discussed above.

Preferable biodegradable core materials include, but are not limited to, naturally derived macromolecules such as collagen, cellulose and hyaluronic acid. Such macromolecules can be cross-linked to reduce the bioresorption rate, or non-cross-linked to allow rapid resorption of the core. Slowly dissolvable thermoplastics may also be utilized to form the biodegradable core, and such materials include, but are not limited to, cellulosic polymers such as hydroxypropylmethylcellulose, e. g., Klucel (S ; polyethyleneoxide, e. g. Polyoxt) ; polyvinylpynolidone; dextran and copolymers of polyethyleneoxide with polypropyleneoxide, e. g., PluronicO3).

As above, the biodegradable core may be conventionally shaped, for instance, as a woven or braided network of monofilaments or multifilaments or wires or as an interconnected network of articulable segments or, e. g. , as a laser-cut or micromachined tube or a coiled structure. For example, the biodegradable core may be constructed of a woven or braided network of biodegradable filaments or fibers, similar to biodegradable sutures. This braided network may then be coated with a biodegradable coating material, as discussed above, which may be a surface erodable material that prevents premature softening of the core structure.

A preferred biodegradable core material is a biodegradable shape memory material, which is typically a polymeric material. Biodegradable shape memory materials are disclosed, e.g., in U.S. Patent No. 6,160,084 . Such materials function similarly to shape memory metallic alloys such as Nitinol® by "remembering" their initial shape. The memory can be triggered by the application of heat to the material configured to a different shape. Thus, when a shape memory polymer is heated above the melting point or glass transition temperature of hard segments in the polymer backbone, the material can be shaped. This (original) shape can be memorized by cooling the shape memory polymer below the melting point or glass transition temperature of the hard segment. When the shaped shape memory polymer is cooled below the melting point or glass transition temperature of a soft segment in the polymer backbone, while the shape is deformed, a new (temporary) shape is fixed. The original shape is recovered by heating the material above the melting point or glass transition temperature of the soft segment but below the melting point or glass transition temperature of the hard segment.

The use of biodegradable shape memory polymers, as with the use of shape memory alloys, is advantageous in that a medical device constructed of such material can be mounted onto a delivery device such as a catheter in compressed shape, and be triggered to return to its memory shape by, e.g., raising its temperature above the transition temperature. This could be accomplished, for example, by contact with body temperature or application of an external heat trigger.

A shape memory biodegradable polymer can be coated with a biodegradable coating material as discussed above. The biodegradable coating material in such an embodiment is also preferably a shape memory polymer that, while preferably being more hydrophobic than the core shape memory polymer, nonetheless exhibits substantially the same transition temperature. Therefore, both the core and the coating material will resume their memory shape upon being subjected to a temperature above the transition temperature.

The core could, for example, be a shape memory biodegradable polymer in the form of a filament or tube that is a laser cut or micromachined to a desired scaffold shape in the deployed, e.g., expanded configuration. Such tube or filament could then be coated with an outer layer of a more hydrophobic surface erodable shape memory biodegradable material with the same or substantially the same transition temperature.

Shape memory biodegradable polymers whose shape change is triggered optically by, for example, application of light to the material are also useful biodegradable materials in the medical devices of the present invention.

One or more additional biodegradable coating layers may be provided on the medical device of the present invention. Any such additional coating layer may be included, for example, to provide added structural features to the medical device, such as enhanced rigidity or flexibility, or to provide enhanced initial biocompatibility with the surrounding and contacting tissue.

Where the core material in any embodiment of the present invention is a polymeric material, whether biodegradable or not, the core can be shaped by any conventional technique, e.g., by extrusion. The core may also be coextruded with any coating material. Alternatively, the core material may be extruded into, e.g., a rod or filament which then may be coated by any known coating method such as, e.g., spray coating, dip coating and, more preferably, coating using fluidized beds or vapor deposition. The coating may also be formed on the core by in-situ polymerization techniques. Conventional shaping and machining procedures, e.g., laser cutting or micromachining, can be employed where the core is a non-polymeric material such as a metallic or ceramic material. These materials may also be coated by spraying, dip coating, fluidized bed coating, vapor deposition and in-situ polymerization.

Either or both of the core or coating material, as well as any additional coating layer, may contain therein or thereon at least one therapeutic agent. While such a therapeutic agent often desirably acts locally, it may also provide substantial systemic benefit.

"Therapeutic agents", "bioactive agents", "pharmaceutically active agents", "pharmaceutically active materials", "drugs" and other related terms may be used interchangeably herein and include genetic therapeutic agents, non-genetic therapeutic agents and cells.

Exemplary non-genetic therapeutic agents include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; and (o)agents that interfere with endogenous vasoactive mechanisms.

Exemplary genetic therapeutic agents include anti-sense DNA and RNA as well as DNA coding for: (a) anti-sense RNA, (b) tRNA or rRNA to replace defective or deficient endogenous molecules, (c) angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors of interest for delivery of genetic therapeutic agents include (a) plasmids, (b) viral vectors such as adenovirus, adenoassociated virus and lentivirus, and (c) non-viral vectors such as lipids, liposomes and cationic lipids.

Cells include cells of human origin (autologous or allogeneic), including stem cells, or from an animal source (xenogeneic), which can be genetically engineered if desired to deliver proteins of interest.

A number of the above therapeutic agents and several others have also been identified as candidates for vascular treatment regimens, for example, as agents targeting restenosis. Such agents are appropriate for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) ACE inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs(6-mercaptopurine), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Several of the above and numerous additional therapeutic agents appropriate for the practice of the present invention are also disclosed in U.S. Patent No. 5,733,925 assigned to NeoRx Corporation, the entire disclosure of which is incorporated herein by reference.

The therapeutic agent may be applied onto the device or any portion thereof, for example, by contacting the device or any portion thereof with a solution or suspension of the therapeutic agent, for example by spraying, dipping, and so forth, followed by evaporating the liquid. The drug may also be incorporated during the processing and/or shaping of any of the polymeric materials used to form the medical device of the present invention provided that the drug is stable at the temperature and pressure conditions required during such processing and/or shaping.

The present invention may be adapted to be utilized with any implantable or insertable medical device including, but not limited to, stents of any shape or configuration, stent grafts, catheters, cerebral aneurysm filler coils, vascular grafts, vena cava filters, heart valve scaffolds and other implantable or insertable medical devices. However, intraluminal stents such as coronary, biliary, urethral, ureteral, tracheal, gastrointestinal and esophageal stents are preferred medical devices.

Particularly preferred intraluminal stents are coronary vascular stents, which may be balloon-expandable or self-expandable. A delivery device is used to deliver a self-expandable stent in a compressed state to a treatment site through a vessel in the body. The flexible nature and reduced radius of the compressed stent enables it to be delivered through relatively small and curved vessels. After the stent is positioned at the treatment site, the delivery device is actuated to release the stent, thereby allowing the stent to self-expand within the body vessel. The delivery device is then detached from the stent and removed from the patient. The stent remains in the vessel at the treatment site as an implant. Stents in accordance with the present invention may also be balloon-expandable. In such stents, a balloon attached to a delivery device such as a catheter is inflated to exert a radial force upon the stent causing it to expand into contact with lumen walls.

The stents may be coiled or patterned as a braided or woven network of fibers or filaments or, for example, as a interconnecting network of articulable segments.

A preferred coronary stent constructed in accordance the present invention:
(a) will maintain adequate rigidity to ensure lumen patency and prevent vessel recoil or abrupt closure for a period of preferably from about three to about six months, more preferably from about two to about four months; (b) will not fragment; (c) will become substantially more flexible, i.e., more biomechanically compatible or compliant with the contacting luminal walls, after about three to about six months and prior to substantial biodegradation or bioresorption; (d) will be fully incorporated into the pannus/neointima; (e) will evoke, if at all, only a benign inflammatory response to metabolites and/or breakdown products without inducing neointimal hyperplasia ; and (g) will preferably be balloon deployable. Such a stent will result in six month and one year restenosis rates equivalent or superior to metallic stents.

A preferred esophageal stent constructed in accordance the present invention: (a) will maintain adequate rigidity for about one to about three months to keep a stricture, e.g. a GERD esophageal stricture, open; (b) will be fully incorporated into mucosal tissue; (c) will become substantially more flexible, i.e., biomechanically compatible or compliant with the contacting luminal walls after about three months; (d) will prevent tissue irritation and will not induce fibrotic/hyperplastic tissue formation induced either by the presence of the device itself or by the metabolites and/or breakdown products; and (e) will preferably be balloon deployable. Such an esophageal stent will result in six month and one year stricture patency rates equivalent to or better than metallic stents.

## Claims

1. An implantable or insertable medical device comprising an inner material comprised of a multifilament core comprising a composite of non-biodegradable wires or filaments and biodegradable wires or filaments, and a biodegradable covering material at least partially covering the inner material; and wherein the covering material provides a diffusion barrier that prevents body fluids from contacting the core material until the covering material has biodegraded.

2. The medical device of claim 1, wherein the inner material is more flexible than the covering material.

3. The medical device of claim 1, wherein the covering material controls the rate at which the inner material becomes flexible upon contact with body fluids.

4. The medical device of claim 3, wherein the covering material is a hydrophobic surface erodable polymer.

5. The medical device of claim 1, wherein at least one of the inner material and the covering material is a polymer.

6. The medical device of claim 5, wherein the polymer is a shape memory biodegradble polymer.

7. The medical device of claim 6, wherein the multifilament core comprises woven or braided filaments.

8. The medical device of one of the claims 1 to 7, wherein the inner material comprises a tubular structure.

9. The medical device of claim 8, wherein the tubular structure is micromachined or laser-cut

10. The medical device of one of the claims 1 to 9, wherein either or both of the inner material and the covering material contains therein or thereon at least one therapeutic agent.

11. The medical device of one of the claims 1 to 10, further comprising one or more additional coating layers.

12. The medical device of claim 11, wherein any of said additional coating layers contains therein or thereon at least one therapeutic agent.

13. The medical device of one of the claims 1 to 12, which is an intraluminal stent.

14. The medical device of claim 13, wherein the intraluminal stent is selected from the group consisting of coronary, biliary, tracheal, gastrointestinal, urethral, ureteral and esophageal stents.

15. The medical device of claim 14, wherein the stent is a self-expandable or balloon-expandable coronary stent.

## Patentansprüche

1. Implantierbares oder einsetzbares Medizinprodukt, umfassend ein Innenmaterial, das aus einem Multifilamentkern besteht, der einen Verbund aus biologisch nicht abbaubaren Drähten oder Filamenten und biologisch abbaubaren Drähten oder Filamenten umfasst, und ein biologisch abbaubares Deckmaterial, das zumindest teilweise das Innenmaterial bedeckt, und wobei das Deckmaterial eine Diffusionssperre bereitstellt, die verhindert, dass Körperflüssigkeiten das Kernmaterial berühren, bis das Deckmaterial biologisch abgebaut ist.

2. Medizinprodukt nach Anspruch 1, wobei das Innenmaterial flexibler als das Deckmaterial ist.

3. Medizinprodukt nach Anspruch 1, wobei das Deckmaterial die Geschwindigkeit steuert, mit der das Innenmaterial bei Kontakt mit Körperflüssigkeiten flexibel wird.

4. Medizinprodukt nach Anspruch 3, wobei das Deckmaterial ein hydrophobes oberflächenerodierbares Polymer ist.

5. Medizinprodukt nach Anspruch 1, wobei mindestens eines, das Innenmaterial oder das Deckmaterial, ein Polymer ist.

6. Medizinprodukt nach Anspruch 5, wobei das Polymer ein biologisch abbaubares Formgedächtnispolymer ist.

7. Medizinprodukt nach Anspruch 6, wobei der Multifilamentkern gewebte oder geflochtene Filamente umfasst.

8. Medizinprodukt nach einem der Ansprüche 1 bis 7, wobei das Innenmaterial eine röhrenförmige Struktur umfasst.

9. Medizinprodukt nach Anspruch 8, wobei die Röhrenstruktur mikrobearbeitet oder lasergeschnitten ist.

10. Medizinprodukt nach einem der Ansprüche 1 bis 9, wobei das Innenmaterial und/oder das Deckmaterial darin oder darauf mindestens einen therapeutischen Wirkstoff enthält bzw. enthalten.

11. Medizinprodukt nach einem der Ansprüche 1 bis 10, ferner umfassend eine oder mehrere zusätzliche Beschichtungsschichten.

12. Medizinprodukt nach Anspruch 11, wobei jede beliebige der zusätzlichen Beschichtungsschichten darin oder darauf mindestens einen therapeutischen Wirkstoff enthält.

13. Medizinprodukt nach einem der Ansprüche 1 bis 12, das ein intraluminaler Stent ist.

14. Medizinprodukt nach Anspruch 13, wobei der intraluminale Stent aus der Gruppe ausgewählt ist, die aus Koronar-, Gallengangs-, Luftröhren-, Gastrointestinal-, Harnröhren-, Harnleiter- und Speiseröhrenstents besteht.

15. Medizinprodukt nach Anspruch 14, wobei der Stent ein selbstexpandierender oder ballonexpandierender Koronarstent ist.

## Revendications

1. Dispositif médical implantable ou insécable comprenant un matériau intérieur composé d'un noyau à filaments multiples comprenant un composite de fils ou filaments non biodégradables et de fils ou filaments biodégadables, et un matériau de couverture biodégradable couvrant au moins partiellement le matériau intérieur ; et dans lequel le matériau de couverture réalise une barrière de diffusion qui empêche des fluides corporels d'entrer en contact avec le matériau du noyau jusqu'à ce que le matériau de couverture se soit biodégradé.

2. Dispositif médical selon la revendication 1, dans lequel le matériau intérieur est plus flexible que le matériau de couverture.

3. Dispositif médical selon la revendication 1, dans lequel le matériau de couverture commande la vitesse à laquelle le matériau intérieur devient flexible lors du contact avec des fluides corporels.

4. Dispositif médical selon la revendication 3, dans lequel le matériau de couverture est un polymère pouvant s'éroder, à surface hydrophobe.

5. Dispositif médical selon la revendication 1, dans lequel, parmi le matériau intérieur et le matériau de couverture, au moins un de ces matériaux est un polymère.

6. Dispositif médical selon la revendication 5, dans lequel le polymère est un polymère biodégradable à mémoire de forme.

7. Dispositif médical selon la revendication 6, dans lequel le noyau à filaments multiples comprend des filaments tissés ou tressés.

8. Dispositif médical selon une des revendications 1 à 7, dans lequel le matériau intérieur comprend une structure tubulaire.

9. Dispositif médical selon la revendication 8, dans lequel la structure tubulaire est micro-usinée ou découpée au laser.

10. Dispositif médical selon une des revendications 1 à 9, dans lequel, parmi le matériau intérieur et le matériau de couverture, soit l'un soit les deux loge(nt) ou porte(n) au moins un agent thérapeutique.

11. Dispositif médical selon une des revendications 1 à 10, comprenant également une ou plusieurs couches de revêtement supplémentaires.

12. Dispositif médical selon la revendication 11, dans lequel l'une quelconque desdites couches de revêtement supplémentaires loge(nt) ou porte(nt) au moins un agent thérapeutique.

13. Dispositif médical selon une des revendications 1 à 12, qui est un stent endoluminal.

14. Dispositif médical selon la revendication 13, dans lequel le stent endoluminal est sélectionné dans le groupe composé de stents coronariens, biliaires, trachéaux, gastro-intestinaux, urétraux, urétéraux et oesophagiens.

15. Dispositif médical selon la revendication 14, dans lequel le stent est un stent coronarien autogonflant ou gonflable par ballonnet.
